# EUROPEAN PATENT SPECIFICATION

(11) **EP 0 655 254 B1**
(45) Date of publication and mention of the grant of the patent: **10.10.2001**
(21) Application number: 94308766.8
(22) Date of filing: 28.11.1994
(51) Int. Cl.: A61M 1/12, A61M 1/10

(54) **Myoplasty device particularly for cardiac assistance**
Myoplastische Vorrichtung insbesondere für Herzunterstützung
Dispositif de myoplastic en particulier pour assistance cardiaque

(30) Priority: 27.11.1993 GB 9324440
(43) Date of publication of application: 31.05.1995
(73) Proprietor: THE UNIVERSITY COURT OF THE UNIVERSITY OF GLASGOW, Glasgow G12 8QQ (GB)
(72) Inventor: Wheatley, David John, Bearsden, Glasgow, G61 1ED (GB)
(74) Representative: Shanks, Andrew

(56) References cited:
- WO-A-94/06395
- US-A- 3 127 846
- US-A- 4 652 265

## Description

This invention relates to a myoplasty device, that is a device which utilises muscle contraction for its operation. The invention has particular application in providing a patient with cardiac assistance utilising the patient's own skeletal muscle.

There have been a number of proposals for providing cardiac assistance utilising a patient's own skeletal muscle. This followed the discovery that adult fast-twitch mammalion skeletal muscle may be conditioned to provide a greatly increased resistance to fatigue such that skeletal muscle may be diverted from its normal function to perform a cardiac assist role. A major advantage of such assistance is that the requirement for cardiac transplantation may be avoided, and the assistance provided by the skeletal muscle may reduce the load on the damaged or diseased heart such that a degree of recovery is possible.

In the British Heart Journal No. 68, 1992, page 333-338, Salmons and Jarvis review the various approaches of providing cardiac assistance from skeletal muscle. In cardiomyoplasty, a skeletal muscle is wrapped around the heart to produce intermittent compression of the patient's left or right ventricle. In aortomyoplasty, the ascending or descending aorta is wrapped directly with skeletal muscle. Alternatively, compression is achieved indirectly by arranging for contraction of the muscle to inflate a cuff placed around a blood vessel. A further approach is to provide an independent structure that is connected to the patient's circulation: skeletal muscle may be configured as an auxiliary pouch-shaped or tube-shaped ventricle by coiling it around a suitable mandrel which is subsequently removed, leaving a skeletal muscle ventricle or a skeletal muscle tube ventricle that can be connected to the circulation. Alternatively, skeletal muscle may be used to energise a mechanical artificial heart or ventricular assist device via a mechanical, hydraulic or electrical link.

In the majority of these approaches the skeletal muscle is coiled or wrapped to define a chamber which is contracted on stimulation of the muscle and dilates when the muscle relaxes. However, the operation of such a system relies upon radial compression of, for example, a pouch or tube by means of a longitudinal contraction of the coiled muscle. This is relatively inefficient and contraction of the coiled muscle also tends to limit the blood supply to the muscle.

International Patent Application W094/06395 (Medtronic) describes a muscle powered cardiac assist device powered by longitudinal contraction of a muscle to actuate a pump. The pump is intended for use with non-blood working fluid, and the fluid enters and exits the pump chamber by a single vent. This document has a publication date subsequent to the priority date of the present application, and therefore falls under the provisions of Art. 54(3) EPC for the Contracting States : DE, FR, GB, IT and NL.

United States Patent No. 4,652,265 (McDougall) describes a myoplasty device comprising anchoring means, and hydraulic motors harnessed to a skeletal muscle in the body of a patient, the hydraulic motors being actuatable on longitudinal contraction of the skeletal muscles to pump hydraulic fluid through hydraulic lines which then serves to drive a blood pump.

It is among the objects of the present invention to provide a myoplasty device adapted to utilise skeletal muscle contractions in an efficient manner and thus overcome the disadvantages associated with existing proposals.

According to the present invention there is provided a myoplasty device comprising: a body including means for anchoring the device adjacent one end of a skeletal muscle in the body of a patient; and blood pumping means mounted on said body and including a blood inlet and a blood outlet and having a portion adapted for attachment to the other end portion of the muscle in the body of the patient, the blood pumping means being directly actuable to pump blood between the inlet and outlet by application of a longitudinal force to said portion on longitudinal contraction of said muscle.

In use, the device may be located in the body of a patient and the anchoring means fixed to, for example, a rib adjacent one end of a skeletal muscle, which may be in its natural location but is more likely to have been moved to a more convenient location in the body. The other end portion of the muscle is then attached to the pump means in such a manner that longitudinal contraction of the muscle will actuate the pump means. In a preferred application, the device provides cardiac assistance. Conveniently such an application utilises a relocated latissimus dorsi muscle which is attached to a rib adjacent the anchoring means at one end and to a portion of the pump means at the other end, the muscle being contracted to actuate the pump means by electrical stimulation with an appropriate pacemaker.

This arrangement thus utilises the muscle in the most efficient manner, that is in longitudinal contraction.

Preferably, the inlet and outlet are adapted to be joined to the atrium and descendeing aorta, respectively.

Preferably, the blood pumping means includes resilient means adapted to restore the blood pumping means to an original condition on release of the longitudinal actuating force. In use, this feature provides for reciprocal operation of the pump means and also serves to extend the relaxed muscle, ready for contraction.

Preferably also the inlet and outlet means include one-way valves.

Preferably also, the blood pumping means includes a chamber having a flexible wall, application of the longitudinal force to a portion of the blood pumping means resulting in deformation of the chamber to provide a part of the pumping cycle. Most preferably, the chamber is arranged such that application of the longitudinal force reduces the volume of the chamber, to expel fluid from the chamber, the chamber then being dilated by a resilient means, said means being suited to pull the muscle back to the resting length.

The chamber may be mounted on an end of the body such that application of said force compresses the chamber against the body. Preferably, the chamber is defined by upper and lower convex walls. A member may be provided for compressing the chamber against the body. Preferably, the member extends over the end of the body and the chamber, such that application of the longitudinal force to end portions of the member compresses the chamber. Conveniently, the member is in the form of a sheet. In the preferred application, this agreement allows the latissimus dorsi muscle to be fashioned into a cone and the open mouth of the muscle cone sutured to a circumferential sewing ring provided at the base of the member, while the tendon at the other end of the muscle is attached to the skeleton of the patient adjacent the anchoring means. This provides a very compact arrangement, such that the device may be located, for example, in the oblique fissure of the left lung.

Inlet and outlet openings may be formed through the sheet to accommodate inlet and outlet conduits.

Preferably also, the body is length adjustable. In use, this permits the pretensioning of the muscle to be adjusted for most efficient operation.

Preferably also, a flexible balloon may be located either within the wall of the pump chamber or between the pump chamber and an external, rigid casing such that fluid may be supplied to the balloon to actuate the pump means. These features allow operation of the device by means of, for example, a small compressor until the skeletal muscle attached to the device has been conditioned and the connections between the muscle and the device become established.

An embodiment of the present invention will now be described, by way of example, with reference to the accompanying drawings in which:
Figure 1 is a sectional view of a myoplasty device in accordance with a preferred embodiment of the present invention, shown attached to a latissimus dorsi muscle; and
Figure 2 is a sectional view of the device of figure 1 shown deployed in the body of a patient.

The drawings illustrate a myoplasty device 10 in accordance with a preferred embodiment of the present invention. The device 10 comprises a two-part body 12 and pump means including a ventricular pumping chamber 14 mounted on the body 12. The body 12 is formed of an inert rigid material and is in the form of a stalk having a narrow end 16 widening somewhat towards the other end 18 to provide mounting for the chamber 14. The length of the body 12, and thus the distance between the ends 16, 18, is variable with provision of a threaded interconnection 19 between the parts of the body 12a and 12b.

The chamber 14 is generally elliptical, the convex base of the chamber defining inlet and outlet openings 20, 21, each of which contains a one-way valve within a sinus 22, 23. Each valve comprises three thin scallop-shaped leaflets suspended from the sinus wall. Valve opening and closure is achieved through passive response to pressure gradients across the valve leaflets, in an analogous manner to the human aortic valve. Conduits 24, 25 extend from the respective openings 20, 21 for connection to appropriate blood vessels.

The ventricular chamber 14 has a convex flexible upper wall 28 which, in its undeformed condition, defines a dome having a form of a half ellipsoid of revolution. A thin sheet of flexible but inextensible material 30 is draped over the top of the chamber 14, being secured to the chamber 14 only at its apex. The sheet 30 extends below the base of the chamber and terminates in a circumferential sewing ring 32.

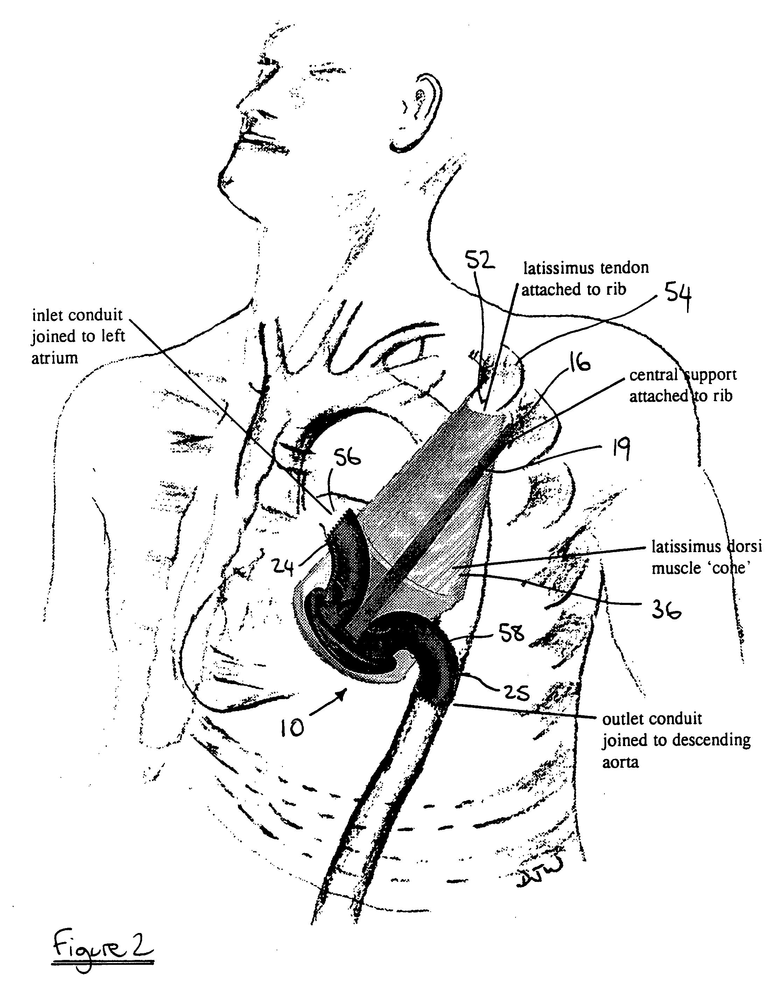

In use, the device 10 is located in the body of a patient 50, as illustrated in Figure 2, to provide a pumping action in parallel with the patient's heart. The device 10 is positioned in the fissure between the upper and lower lobes of the left lung. For its operation, the device 10 relies upon the latissimus dorsi muscle 36 which has been previously relocated within the body of the patient, the neurovascular bundle to the muscle 36 being preserved. The latissimus dorsi tendon 52 at one end of the muscle 36 is attached to a rib 54 close to the narrow end 16 of the body, the body 12 also being fixed to the rib. The remainder of the muscle is fashioned into a cone and attached to the sewing ring 32. The threaded connection 19 is utilised to adjust the length of the body 12 to provide the desired resting muscle length. The inlet conduit 24 is connected to the left atrium 56, while the outlet conduit 25 returns the blood to the descending aorta 58.

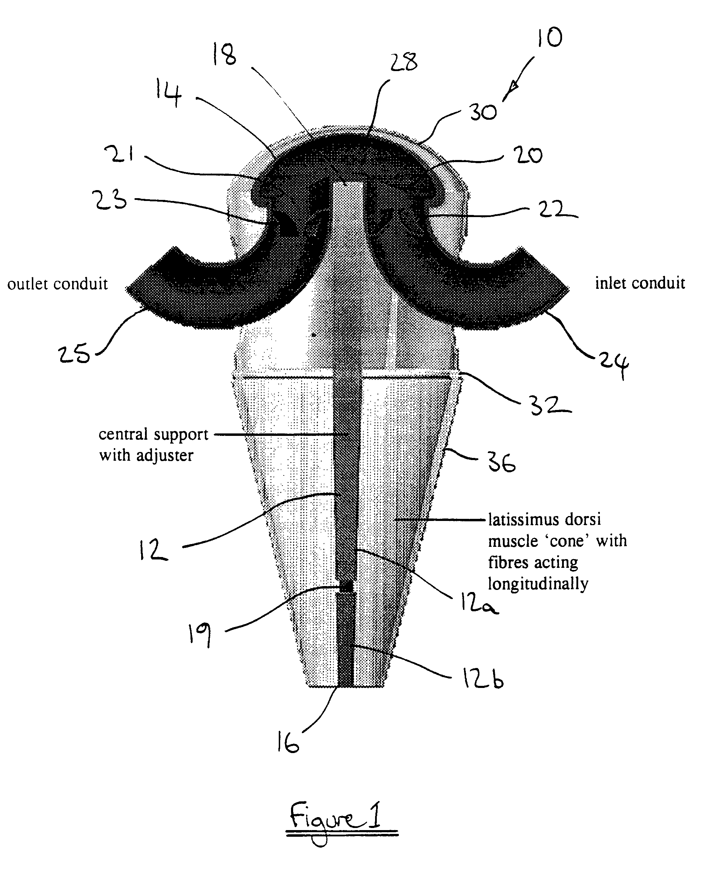

As the muscle cone 36 contracts, under electrical stimulation provided by an appropriate pacemaker (not shown), the sheet is pulled downwards (as viewed in Figure 1) and the ventricular chamber 14 collapses. The upper chamber wall 28 is shaped such that the chamber 14 will collapse in a uniform and predictable manner and also provides the restoring force to pull the collapsed chamber 14, and the contracted muscle cone, back to its original state during "diastole". Thus, in normal operation, there is no requirement to provide any additional springs and the like. The use of the sheet 30 to collapse the chamber 14 provides a rolling hinge type mechanism facilitating a collapse of the dome in a smooth and predictable manner with the associated bending stresses distributed over a wide area, rather than being concentrated at any one localised point, with subsequent risk of fatigue failure. Also, those of skill in the art will realise that the internal configuration of the chamber 14, the inlets and outlets 20, 21, the valve sinuses 22, 23 and the conduits 24, 25, must be carefully designed to enable the chamber to be fully washed out, avoiding stagnation areas. It will also be clear that the device should be formed of inert medical grade materials avoiding unnecessary seams and the like, and to this end the flexible upper wall 28 of the chamber 14, valve openings 20, 21, the valves and the valve sinuses 22, 23 are formed from a seamless single continuous sheet of polyurethane.

In operation, the geometry of the device is such that the muscle 36 acts along its own length in a straight line, as it does in its natural configuration. This provides for more efficient use of the muscle. Further, the geometry of the device enables a large ejection fraction to be achieved with only a modest change in muscle length. The device 10 may function asynchronously with the heart, the rate being determined by optimal performance of the skeletal muscle.

A balloon (not shown) is provided either within the wall of the pump chamber or between the pump chamber and an external, rigid casing. A small access port allows a suitable tube to pass from the balloon to an external compressor (not shown) of the type currently used, for example, with intra-aortic balloon pumps. Thus, by inflating and deflating the balloon, the ventricular chamber 14 can be collapsed and distended.

The compressor may be utilised to actuate the device 10 for a period after insertion of the device 10 into the patient's body. During this time the muscle 36 may be conditioned to withstand the different operating conditions that it will experience and also allows the connection between the muscle 36 and the sewing ring 32 to become established.

From the above description it will be clear to those of skill in the art that the device 10 provides a simple yet efficient means for providing cardiac assistance. This is due to a large extent to the configuration of the device which allows the skeletal muscle to be used as normal, that is by longitudinal contraction, while the use of a resilient chamber 14 provides a simple means for extending the contracted muscle.

It will also be clear to those of skill in the art that the above-described embodiment is merely exemplary of the present invention and that various modifications and improvements may be made thereto without departing from the scope of the invention.

## Claims

1. A myoplasty device (10) comprising: a body including means (16) for anchoring the device adjacent one end of a skeletal muscle in the body of a patient; and blood pumping means mounted on said body and including a blood inlet and a blood outlet (20, 21) and having a portion (30, 32) adapted for attachment to the other end portion of the muscle in the body of the patient (14), the blood pumping means being directly actuable to pump blood between the inlet and outlet (20,21) by application of a longitudinal force to said portion (30, 32) on longitudinal contraction of the muscle.

2. The device of claim 1 wherein the inlet and outlet (20, 21) are adapted to be joined to the atrium and to the descending aorta, respectively.

3. The device of any of the preceding claims wherein the blood pumping means includes resilient means (28) adapted to restore the blood pumping means to an original condition on release of the longitudinal actuating force.

4. The device of any of the preceding claims wherein the inlet and outlet means include one-way valves (22, 23).

5. The device of any of the preceding claims wherein the blood pumping means includes a chamber (14) having a flexible wall (28), application of the longitudinal force to a portion of the blood pumping means resulting in deformation of the chamber (14) to provide a part of the pumping cycle.

6. The device of claim 5 wherein the chamber (14) is arranged such that application of the longitudinal force reduces the volume of the chamber, to expel fluid from the chamber, the chamber then being dilated by a resilient means, said means being suited to pull the muscle back to the resting length.

7. The device of claim 5 or 6 wherein the chamber (14) is located on an end of the body (18) such that application of said force compresses the chamber against the body.

8. The device of claim 7 wherein an upper wall and a lower wall of the chamber each define a convex surface.

9. The device of claim 7 or 8 wherein a member (30) is provided for compressing the chamber against the body (18).

10. The device of claim 9 wherein the member (30) extends over the end of the body and the chamber (14), such that application of the longitudinal force to end portions of the member compresses the chamber.

11. The device of claim 10 wherein the member is in the form of a sheet (30).

12. The device of claim 11 in which openings are provided in the sheet (30) to accommodate inlet and outlet conduits (24, 25) extending from the chamber (14).

13. The device of any of claims 5 to 12 wherein a flexible balloon is located within the wall of the pump chamber or between the pump chamber and an external, rigid casing such that fluid may be supplied to the balloon to actuate the blood pumping means.

14. The device of any of the preceding claims wherein the body (12) is length adjustable.

## Patentansprüche

1. Myoplastiegerät (10), das folgendes aufweist: einen Körper, der Mittel (16) zur Verankerung des Geräts angrenzend an ein Ende eines Skelettmuskels im Körper eines Patienten einschließt, und Blutpumpmittel, die an dem Körper angebracht sind und einen Bluteinlaß und einen Blutauslaß (20, 21) einschließen und einen Abschnitt (30, 32) haben, der für die Befestigung an dem anderen Endabschnitt des Muskels im Körper des Patienten (14) geeignet ist, wobei die Blutpumpmittel direkt betätigt werden können, um Blut zwischen dem Einlaß und dem Auslaß (20, 21) zu pumpen, wenn bei der Längskontraktion des Muskels eine Längskraft auf den Abschnitt (30, 32) ausgeübt wird.

2. Gerät nach Anspruch 1, bei dem der Einlaß und der Auslaß (20, 21) dafür geeignet sind, mit dem Vorhof bzw. der absteigenden Aorta verbunden zu werden.

3. Gerät nach einem der vorhergehenden Ansprüche, bei dem die Blutpumpmittel elastische Mittel (28) einschließen, die in der Lage sind, die Blutpumpmittel wieder in einen Ausgangszustand zurückzuführen, wenn die in Längsrichtung wirkende Kraft aufgehoben wird.

4. Gerät nach einem der vorhergehenden Ansprüche, bei dem die Einlaß- und die Auslaßmittel Einwegventile (22, 23) einschließen.

5. Gerät nach einem der vorhergehenden Ansprüche, bei dem die Blutpumpmittel eine Kammer (14) mit einer flexiblen Wand (28) einschließen, wobei das Ausüben der Längskraft auf einen Abschnitt der Blutpumpmittel zur Verformung der Kammer (14) führt, um einen Teil des Pumpzyklusses bereitzustellen.

6. Gerät nach Anspruch 5, bei dem die Kammer (14) derartig angeordnet ist, daß durch das Ausüben der Längskraft das Volumen der Kammer verringert wird, um Fluid aus der Kammer auszustoßen, worauf die Kammer dann durch elastische Mittel gedehnt wird, wobei die Mittel geeignet sind, den Muskel zurück auf die Ruhelänge zu schieben.

7. Gerät nach Anspruch 5 oder 6, bei dem die Kammer (14) an einem Ende des Körpers (18) angeordnet ist, derartig, daß durch das Ausüben der Kraft die Kammer gegen den Körper gedrückt wird.

8. Gerät nach Anspruch 7, bei dem eine obere Wand und eine untere Wand der Kammer jeweils eine konvexe Fläche definieren.

9. Gerät nach Anspruch 7 oder 8, bei dem ein Glied (30) bereitgestellt wird, um die Kammer gegen den Körper (18) zusammenzudrücken.

10. Gerät nach Anspruch 9, bei dem sich das Glied (30) über das Ende des Körpers und die Kammer (14) erstreckt, derartig, daß durch das Ausüben der Längskraft auf die Endabschnitte des Gliedes die Kammer zusammengedrückt wird.

11. Gerät nach Anspruch 10, bei dem das Glied die Form einer dünnen Lage (30) hat.

12. Gerät nach Anspruch 11, bei dem in der dünnen Lage (30) Öffnungen bereitgestellt werden, um Einlaß- und Auslaßleitungen (24, 25) aufzunehmen, die von der Kammer (14) ausgehen.

13. Gerät nach einem der Ansprüche 5 bis 12, bei dem sich innerhalb der Wand der Pumpenkammer oder zwischen der Pumpenkammer und einem äußeren, starren Gehäuse ein flexibler Ballon befindet, derartig, daß dem Ballon Fluid zugeführt werden kann, um die Blutpumpmittel zu betätigen.

14. Gerät nach einem der vorhergehenden Ansprüche, bei dem der Körper (12) in der Länge verstellbar ist.

## Revendications

1. Dispositif de myoplastie (10), comprenant: un corps englobant un moyen (16) pour fixer le dispositif près d'une extrémité d'un muscle squelettique dans le corps d'un patient; un moyen pour pomper le sang monté sur ledit corps et englobant un orifice d'admission du sang et un orifice de sortie du sang (20, 21) et comportant une partie (30, 32) destinée à être fixée sur l'autre partie d'extrémité du muscle dans le corps du patient (14), le moyen de pompage du sang pouvant être actionné directement pour pomper le sang entre l'orifice d'admission et l'orifice de sortie (20, 21) par application d'une force longitudinale à ladite partie (30, 32) lors de la contraction longitudinale du muscle.

2. Dispositif selon la revendication 1, dans lequel l'orifice d'admission et l'orifice de sortie (20, 21) sont destinés à être respectivement reliés au vestibule et à l'aorte descendante.

3. Dispositif selon l'une quelconque des revendications précédentes, dans lequel le moyen de pompage du sang englobe un moyen élastique (28) destiné à ramener le moyen de pompage du sang vers un état d'origine lors de la suppression de la force d'actionnement longitudinale.

4. Dispositif selon l'une quelconque des revendications précédentes, dans lequel les moyens d'orifice d'admission et de sortie englobent des valves anti-reflux (22, 23).

5. Dispositif selon l'une quelconque des revendications précédentes, dans lequel le moyen de pompage du sang englobe une chambre (14) comportant une paroi flexible (28), l'application de la force longitudinale à une partie du moyen de pompage du sang entraînant une déformation de la chambre (14) pour établir une partie du cycle de pompage.

6. Dispositif selon la revendication 5, dans lequel la chambre (14) est agencée de sorte que l'application de la force longitudinale réduit le volume de la chambre, pour expulser le fluide de la chambre, la chambre étant dilatée par un moyen élastique, ledit moyen étant destiné à tirer le muscle vers l'arrière vers la longueur de repos.

7. Dispositif selon les revendications 5 ou 6, dans lequel la chambre (14) est agencée sur une extrémité du corps (18), de sorte que l'application de ladite force comprime la chambre contre le corps.

8. Dispositif selon la revendication 7, dans lequel une paroi supérieure et une paroi inférieure de la chambre définissent chacune une surface convexe.

9. Dispositif selon les revendications 7 ou 8, comportant un élément (30) servant à comprimer la chambre contre le corps (18).

10. Dispositif selon la revendication 9, dans lequel l'élément (30) s'étend au-dessus de l'extrémité du corps et de la chambre (14) de sorte que l'application de la force longitudinale aux parties d'extrémité de l'élément comprime la chambre.

11. Dispositif selon la revendication 10, dans lequel l'élément a la forme d'une feuille (30).

12. Dispositif selon la revendication 11, comportant des ouvertures dans la feuille (30) pour recevoir les conduites d'entrée et de sortie (24, 25) s'étendant à partir de la chambre (14).

13. Dispositif selon l'une quelconque des revendications 5 à 12, dans lequel un ballon flexible est agencé à l'intérieur de la paroi de la chambre de pompage ou entre la chambre de pompage et une enceinte externe rigide, de sorte que le fluide peut être amené vers le ballon pour actionner le moyen de pompage du sang.

14. Dispositif selon l'une quelconque des revendications précédentes, dans lequel la longueur du corps (12) peut être ajustée.
